# EUROPÄISCHE PATENTSCHRIFT

(11) **EP 0 984 802 B1**
(45) Veröffentlichungstag und Bekanntmachung des Hinweises auf die Patenterteilung: **24.09.2003**
(21) Anmeldenummer: 98958880.1
(22) Anmeldetag: 29.10.1998
(51) Int. Cl.: A61M 1/36, A61M 5/36, A61M 1/00

(54) **VORRICHTUNG ZUR FILTRATION UND ENTGASUNG VON KÖRPERFLÜSSIGKEITEN, INSBESONDERE BLUTFILTER**
DEVICE FOR FILTERING AND DEGASSING BODILY FLUIDS, ESPECIALLY A BLOOD FILTER
DISPOSITIF POUR LA FILTRATION ET LE DEGAZAGE DE FLUIDES CORPORELS, NOTAMMENT FILTRE POUR LE SANG

(30) Priorität: 12.11.1997 DE 19750062
(43) Veröffentlichungstag der Anmeldung: 15.03.2000
(73) Patentinhaber: Jostra AG, 72145 Hirrlingen (DE)
(72) Erfinder: SPRANGER, Martin, D-72119 Ammerbuch (DE)
(74) Vertreter: Otten, Hajo, Dr.-Ing.
(86) Internationale Anmeldenummer: EP9806860
(87) Internationale Veröffentlichungsnummer: WO99024087

(56) Entgegenhaltungen:
- EP-A- 0 315 022
- EP-A- 0 743 071
- DE-A- 3 541 521
- US-A- 4 190 426
- US-A- 5 304 164
- US-A- 5 458 567

## Beschreibung

Die Erfindung betrifft eine Vorrichtung zur Filtration und Entgasung von Körperflüssigkeiten, insbesondere Blutfilter, mit zumindest zwei voneinander durch ein Filtermedium getrennten Filterkammern, die vorzugsweise in einem gemeinsamen Gehäuse angeordnet sind, wobei die erste Filterkammer mit einem Zulaufanschluß für zu filtrierende Körperflüssigkeit und die zweite Filterkammer mit einem Ablaufanschluß für filtrierte Körperflüssigkeit verbunden ist.

Eine derartige Vorrichtung ist aus der DE 35 41 521 C2 bekannt.

Die bekannte Vorrichtung ist ein Blutfilter, dessen Zulaufanschluß in Betriebsstellung senkrecht von unten an die erste Filterkammer angeschlossen ist. Der Zulaufanschluß führt in ein Steigrohr, das die zylindrische erste Filterkammer zu etwa 2/3 ihrer Höhe durchsetzt. Oben aus dem Steigrohr, das als Überlauf ausgebildet ist, tritt das zu filternde Blut aus und fällt außen am Steigrohr in einen Sinkraum.

Bei diesen Überlaufen scheidet sich in dem Blut gelöstes Gas in Form von Gasbläschen aus, die nach oben treiben. Oben in der ersten Filterkammer ist ein Gasaustritt, über den die erste Filterkammer entlüftet wird.

Das Steigrohr ist von einem hohlzylindrischen Filtermedium umgeben, an das sich außen eine hohlzylindrische zweite Filterkammer anschließt, die an ihrem oberen Ende mit einer Gasaustrittsöffnung zur Entlüftung der zweiten Filterkammer versehen ist.

In Betriebsstellung unten ist die zweite Filterkammer mit einem horizontal abgehenden Ablaufanschluß verbunden, durch den das gefilterte Blut aus dem bekannten Blutfilter austritt.

Das bekannte Blutfilter wird für extrakorporale Blutkreisläufe eingesetzt, bei denen Blut vor der Rückgabe in den Patienten filtriert und von Gasblasen befreit wird. Dieser Schritt darf die Blutzellen nicht schädigen und keine Gerinnungsmechanismen auslösen. Ferner sind Blutverluste zu vermeiden. Deshalb wird bei dem bekannten Filter die Abscheidung von Teilchen und Aggregaten in Form einer dead-end-Filtration durchgeführt, was jedoch den Nachteil aufweist, daß sich die zurückgehaltenen Bestandteile allmählich in dem Filter akkumulieren. Bei dem bekannten Filter sammeln sich diese Bestandteile in dem Sinkraum, wodurch das Filtermedium sich allmählich von unten nach oben fortschreitend durch sedimentierende Teilchen und Aggregate zusetzt.

Durch dieses Zusetzen kann es bei einem in einen extrakorporalen Blutkreislauf eingeschalteten Blutfilter zu einer unerwünschten Minderung des Volumenflusses des filtrierten Vollblutes bis hin zu einer vollständigen Unterbrechung des Kreislaufes sowie zu einem unzulässigen Druckanstieg über dem Filter kommen, was unerwünschte Wirkung auf die Blutbestandteile hat.

Zu diesem Zweck ist es bekannt, daß bei einem Blutfilter der eingangs genannten Art ein Bypass mit z.B. zwei Y-Stücken aufgebaut wird, wobei die beiden Arme der beiden Y-Stücke mit dem Blutfilter bzw. einer Bypassleitung verbunden werden. Der jeweilige Stiel der Y-Leitung bildet dann den Zulauf bzw. den Ablauf für die Anordnung aus Blutfilter und Bypass. In die Bypassleitung ist ein Absperrventil eingeschaltet, wobei es auch bekannt ist, die Bypassleitung durch eine Schlauchklemme zu unterbrechen.

Wenn sich jetzt das Blutfilter unzulässig zugesetzt hat, wird die Bypassleitung geöffnet, so daß der Blutkreislauf an dem Blutfilter vorbei aufrechterhalten werden kann.

Diese spezielle Anordnung des Blutfilters mit Bypassleitung erfordert größeren apparativen Aufwand sowie besondere Reinigungsmaßnahmen und ist daher von Nachteil. Ein weiterer Nachteil bei dieser Bypassleitung besteht darin, daß sich in der Bypassleitung oberhalb des Absperrventiles bzw. der Schlauchklemme koaguliertes Blut ansammeln kann, das bei einem plötzlichen Öffnen der Bypassleitung als Pfropfen in den Blutkreislauf gelangen und ungünstigstenfalls zu Embolien führen kann.

Ein weiterer Nachteil bei dem bekannten Blutfilter ist darin zu sehen, daß durch die beiden getrennten Entlüftungsöffnungen iterative Handgriffe erforderlich sind, um das Blutfilter vollständig zu entlüften. Eine derartige Entlüftung ist jedoch vor dem Einsatz des Blutfilters in den extrakorporalen Blutkreislauf erforderlich, um zu verhindern, daß Gasblasen in den Blutkreislauf gelangen, was zu den bekannten negativen Effekten führen kann.

Insgesamt gibt es bei dem bekannten Blutfilter also große Handhabungs- und Sterilitätsprobleme, die mit der Bypassleitung sowie den beiden Entlüftungsöffnungen verbunden sind. Weiterhin zeigt das bekannte Blutfilter Sicherheitsrisiken im Einsatz, die sich aus der Bypassleitung sowie der Sedimentierung der abgeschiedenen Teilchen und Aggregate ergeben.

Allgemein ist bei dem bekannten Blutfilter noch sein voluminöser Aufbau von Nachteil, der insbesondere daraus resultiert, daß der Entlüftungsraum der inneren, ersten Filterkammer oberhalb des Steigrohres sowie der zweiten Filterkammer angeordnet ist.

Aus der DE 196 20 591 ist eine kompaktere Vorrichtung zum Entfernen von Gasen aus Flüssigkeiten bekannt, die das aus dem oben zitierten Blutfilter bekannte Prinzip der Entgasung durch Überlauf verwendet. Die Überlaufverbindung ist hier zwischen einer zylindrischen inneren und einer hohlzylindrischen äußeren Filterkammer vorgesehen, wobei der den Überlauf gewährleistende Strömungsraum mit einem hydrophoben Filter versehen ist, das das austretende Gas nach oben abführt, das Blut jedoch zurückhält. Das Blut selbst wird bei dieser Vorrichtung nicht durch ein Filtermedium geführt, es handelt sich hier also nicht um ein Blutfilter.

Ein weiteres, sehr spezielles und nicht universell einsetzbares Filter für Hämofiltration oder Plasmapherese, bei dem über dem Filtermedium also ein hoher Druck abfällt, ist aus der EP 0 076 421 bekannt. Dieses Filter umfaßt ein inneres sowie ein äußeres Faserbündel, das jeweils mit einem Einlaß- bzw. Auslaßanschluß verbunden ist. Ferner gibt es einen Filtratauslaß, es handelt sich also nicht um dead-end-Filter sondern um cross-flow-Filter mit entsprechendem Blutverlust.

Zwischen dem Einlaß und dem Auslaß ist eine Art Überdruckventil angeordnet, das automatisch eine Verbindung zwischen dem Einlaß und dem Auslaß herstellt, wenn der Druckabfall über den in Reihe geschalteten Faserbündeln zu hoch wird.

Aus Sicherheitsgründen ist dieses Filter nur bedingt für den Einsatz in extrakorporalen Blutkreisläufen geeignet. Ein Nachteil des Filters besteht darin, daß das Ansprechen des Überdruckventiles nicht sichtbar ist, so daß das Bedienungspersonal nicht erkennen kann, ob der Filter verstopft ist oder noch zufriedenstellend arbeitet. Ferner birgt das Überdruckventil das Risiko eines ungewollten Öffnens bzw. von Leckströmen; in bestimmten Druckbereichen öffnet es sich nämlich nur partiell, wobei infolge von Schwankungen der Eigenschaften der verwendeten Materialien sowie infolge von Alterungs- und Belastungsprozessen der Öffnungsdruck nicht reproduzierbar einzustellen ist.

Ein weiterer Nachteil dieses Filters besteht darin, daß es "taktet"; nach dem Öffnen infolge eines unzulässig hohen Druckanstieges über dem Filtermedium fällt dieser Druck nämlich in der Regel wieder ab, so daß das Überdruckventil wieder schließt, was dann erneut zu einem Druckaufbau führt, der das Überdruckventil wieder öffnet.

Ausgehend von diesem Stand der Technik ist es Aufgabe der vorliegenden Erfindung, die eingangs erwähnte Vorrichtung derart weiterzuentwickeln, daß bei einfachem konstruktivem Aufbau und kleiner Bauweise die obigen Nachteile vermieden werden und sich insbesondere eine leichte und zuverlässige Handhabung mit einer für den Patienten sicheren Funktion ergibt.

Bei der eingangs erwähnten Vorrichtung wird diese Aufgabe erfindungsgemäß dadurch gelöst, daß eine beiden Filterkammern gemeinsame Be- und Entlüftungseinrichtung vorgesehen ist, die wahlweise und separat mit der ersten bzw. zweiten Filterkammer verbindbar ist.

Die der Erfindung zugrunde liegende Aufgabe wird auf diese Weise vollkommen gelöst.

Der Erfinder der vorliegenden Anmeldung hat nämlich erkannt, daß es insbesondere durch noch zu beschreibende konstruktive Maßnahmen möglich ist, für beide Filterkammern eine gemeinsame Entlüftung vorzusehen, so daß beide Kammern separat und wahlweise entlüftbar sind. Auf diese Weise läßt sich lediglich durch Manipulation an einem z.B. Ventil der neuen Vorrichtung zuerst eine sich schneller mit Körperflüssigkeit füllende äußere Filterkammer und dann durch einfaches Verschwenken eine sich danach vollständig füllende innere Filterkammer entlüften. Die gemeinsame Be- und Entlüftungseinrichtung hat jedoch neben der einfachen Handhabung auch konstruktive Vorteile, es ist lediglich für die Abdichtung einer einzigen Entlüftungseinrichtung zu sorgen. Ein weiterer Vorteil liegt darin, daß die Kontaminationsgefahr an der oder durch die Belüftung auf eine einzige Be- und Entlüftungseinrichtung beschränkt wird.

In einer Weiterbildung ist es bevorzugt, wenn zwischen dem Zulauf- und dem Ablaufanschluß, die vorzugsweise zueinander benachbart am in Betriebsstellung tiefsten Punkt der Vorrichtung angeordnet sind, ein Verbindungselement vorgesehen ist, über das Zulauf- und Ablaufanschluß unter Umgehung des Filtermediums wahlweise direkt miteinander verbindbar sind.

Diese Maßnahme erhöht die Betriebssicherheit der neuen Vorrichtung in großem Umfang, denn durch sie wird ein wahlweise schaltbarer Bypass gebildet, so daß auf die im Stand der Technik erforderliche Bypassleitung mit den damit verbundenen Nachteilen und Problemen verzichtet werden kann. Zum einen reduziert sich der apparative Aufwand durch die "integrierte Bypassleitung", wobei zum anderen die Ansammlung von koaguliertem Blut und Aggregaten vermieden wird, so daß die Emboliegefahr deutlich herabgesetzt wird.

Damit löst diese Maßnahme aber auch separat, also ohne die gemeinsame Be- und Entlüftungseinrichtung, die der Erfindung zugrunde liegende Aufgabe bei dem eingangs genannten, gattungsbildenden Blutfilter und ist für sich genommen erfinderisch.

Weiter ist es bevorzugt, wenn in der ersten Filterkammer Strömungsleitmittel vorgesehen sind, die die eintretende Körperflüssigkeit in zumindest einen ersten, das Filtermedium in Betriebsstellung von unten und zumindest einen weiteren, das Filtermedium etwa mittig anströmenden Teilstrom aufteilt.

Bei dieser Maßnahme ist von Vorteil, daß ein allmählich von unten ansteigendes Zusetzen des Filtermediums verhindert wird, denn der im Stand der Technik nicht vorgegebene, das Filtermedium von unten anströmende erste Teilstrom wirbelt die Sedimente wieder auf, so daß die gesamte Filterfläche für die Filtrierung der Körperflüssigkeit zur Verfügung steht. Der Erfinder der vorliegenden Anmeldung hat erkannt, daß durch diese Maßnahme eine etwa gleichmäßige Verteilung der Filterrückstände in der ersten Filterkammer erreicht wird, wo diese Teilchen verwirbelt werden, so daß wider Erwarten die Filterwirkung sowie die Durchlaßmenge des Filters sich im Laufe der Zeit deutlich weniger reduziert als bei einem gattungsgemäßen Blutfilter.

Damit löst aber auch diese Maßnahme die der Erfindung zugrunde liegende Aufgabe für sich genommen bei der gattungsbildenden Vorrichtung, also ohne die gemeinsame Be- und Entlüftungseinrichtung sowie den integrierten Bypass. Auch diese Maßnahme ist bei dem gattungsbildenden Blutfilter also für sich genommen erfinderisch.

Insbesondere die Kombination der drei insoweit beschriebenen Maßnahmen führt jedoch zu einem sehr einfach zu handhabenden und sehr sicher arbeitenden Filter für Körperflüssigkeiten, das zudem konstruktiv einfach aufgebaut ist und eine kleine, kompakte Bauweise aufweist.

In einer Weiterbildung ist es dann bevorzugt, wenn das Gehäuse eine prismatische Form mit vorzugsweise quadratischem Querschnitt aufweist und an einer Ecke des Gehäuses die Be- und Entlüftungseinrichtung angeordnet ist.

Bei dieser Maßnahme ist von Vorteil, daß sich das Gas in der im Betriebszustand oben gebildeten Spitze ansammelt, so daß eine effiziente Be- und Entlüftung insbesondere auch beim Wiederbefüllen möglich wird. Ein weiterer Vorteil liegt in der kompakten Anordnung sowie der großen, für die Filterwirkung zur Verfügung stehenden quadratischen Grundfläche der neuen Vorrichtung.

Weiter ist es bevorzugt, wenn die Be- und Entlüftungseinrichtung einen drehbar gelagerten Hohlzylinder umfaßt, dessen nach außen führender Innenkanal durch Drehen des Hohlzylinders wahlweise mit einer der beiden Filterkammern verbindbar ist.

Diese Maßnahme ist konstruktiv von Vorteil, ein einziger, an dem Gehäuse drehbar gelagerter Hohlzylinder ermöglicht die separate und wahlweise Entlüftung beider Filterkammern. Ein weiterer Vorteil dieser Anordnung liegt darin, daß sie einfach zu sterilisieren und problemlos in einer Ecke des Gehäuses anzuordnen ist.

Weiter ist es bevorzugt, wenn der Ablaufanschluß ein Rohrstück umfaßt, das sich in der ersten Filterkammer vorzugsweise als Strömungsleitmittel quer über dem Zulaufanschluß erstreckt.

Auch diese Maßnahme ist konstruktiv von Vorteil, das Rohrstück dient zum einen als Ablauf für die filtrierte Körperflüssigkeit und zum anderen zur Aufteilung der einströmenden Körperflüssigkeit, so daß diese gleichmäßig auf das Filtermedium verteilt wird, wobei vorzugsweise weiter für die Anströmung des Filtermediums von unten gesorgt werden kann.

In einer Weiterbildung ist das Rohrstück drehbar gelagert und weist eine nach außen führende Bohrung auf, deren innere Öffnung durch Drehen wahlweise mit der zweiten Filterkammer oder dem Zulaufanschluß verbindbar ist, so daß das Rohrstück als Verbindungselement wirkt.

Auch diese Maßnahme ist konstruktiv von Vorteil, das Rohrstück erfüllt nämlich als weitere Funktion die des integrierten Bypasses. Ein weiterer Vorteil liegt darin, daß die Bohrung des Rohrstückes sowohl im Filterbetrieb als auch im Bypassbetrieb von der Körperflüssigkeit durchströmt wird, so daß sich kein koagulierendes Blut oder sonstige Aggregate ansammeln können, die beim Umschalten auf Bypass-Betrieb zu einer Emboliegefahr führen können.

Allgemein ist es bevorzugt, wenn die zweite Filterkammer zumindest zwei parallele, flach ausgebildete Teilkammern umfaßt, die in der ersten Filterkammer angeordnet sind und von dieser beidseitig durch Filtermedium getrennt sind.

Diese Maßnahme ermöglicht einen kompakten Aufbau der neuen Vorrichtung, da sie auf kleinem Raum eine große Fläche des Filtermediums für die Filterwirkung zur Verfügung stellt. Ferner können durch derartige, parallel zueinander angeordnete Teilkammern der zweiten Filterkammer günstige Strömungsverhältnisse bewirkt werden, durch die die Sedimente aufgewirbelt werden, so daß auch durch diese Maßnahme insgesamt das Zusetzen des Filtermediums effektiv verhindert wird.

Dabei ist es dann bevorzugt, wenn das Filtermedium als Vliesoder Siebgewebe ausgebildet und so an Tragplatten oder -rahmen befestigt ist, daß keine Gasblasen eingefangen werden, wobei das Filtermedium vorzugsweise durch in Betriebsstellung der Vorrichtung vertikal ausgerichtete Stützelemente, vorzugsweise Stege oder Noppen, unterstützt wird.

Diese Maßnahme ermöglicht ein schnelles Entlüften der neuen Vorrichtung, da sich keine Gasblasen an Rippen oder sonstigen Unterstützungselementen für das Filter verfangen können. Aus der Praxis ist es bekannt, daß bei der Entlüftung von Filtern immer wieder gegen das Gehäuse geklopft wird, um die an beliebigen Stützelementen innen im Filter festsitzenden Gasblasen zu lösen. Der Erfinder der vorliegenden Anmeldung hat nunmehr erkannt, daß durch die hier beschriebenen Maßnahmen auf konstruktive Weise bereits verhindert wird, daß sich Gasblasen überhaupt im Filter ansammeln. Hierzu trägt auch die Verwendung eines flachen Vlies- oder Siebgewebes bei, das im Gegensatz zu plissierten Filtermedien, wie sie bei der eingangs erwähnten DE 35 41 521 C2 verwendet werden, nicht nur die Ansammlung von Gasblasen im oder am Filtermedium sondern auch das Ansammeln von Aggregaten oder sonstigen Sedimenten effektiv verhindert. Durch die Anströmung des Filtermediums von unten sowohl beim ersten Befüllen als auch im Filterbetrieb wird nämlich durch den von unten aufsteigenden Teilstrom der Körperflüssigkeit dafür gesorgt, daß ggf. anhaftende Sedimente oder Gasblasen effektiv von der Filterfläche entfernt werden. Hierdurch wird für eine gute Entlüftung sowie eine effektive Filterwirkung gesorgt.

Schließlich ist es noch bevorzugt, wenn die Strömungsleitmittel Strömungsbleche umfassen, die parallel zu Schmalseiten des Gehäuses angeordnet sind.

Auch diese Maßnahme trägt zu einer effektiven Verteilung der Teilströme in der neuen Vorrichtung bei, wodurch die Körperflüssigkeit sowohl beim ersten Befüllen als auch im Betrieb derart in der neuen Vorrichtung verwirbelt wird, daß trotz der oben beschriebenen Maßnahmen im Filter anhaftende Gasblasen und/oder Sedimente abgelöst werden.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist in der Zeichnung dargestellt und wird in der nachfolgenden Beschreibung näher erläutert. Es zeigen:
- Fig. 1: eine perspektivische Ansicht einer längs geschnittenen Ausführungsform der Vorrichtung;
- Fig. 2: eine vergrößerte Detailansicht der Be- und Entlüftungseinrichtung der Vorrichtung aus Fig. 1 in einem ersten Betriebszustand;
- Fig. 3: die Be- und Entlüftungseinrichtung aus Fig. 2 in einem zweiten Betriebszustand;
- Fig. 4: eine vergrößerte Detailansicht des Verbindungselementes der Vorrichtung aus Fig. 1 in einem ersten Betriebszustand;
- Fig. 5: in einer Ansicht wie Fig. 4 das Verbindungselement in einem zweiten Betriebszustand;
- Fig. 6: eine Draufsicht auf die Vorrichtung aus Fig. 1 im Querschnitt; und
- Fig. 7: eine perspektivische Ansicht der Vorrichtung aus Fig. 6.

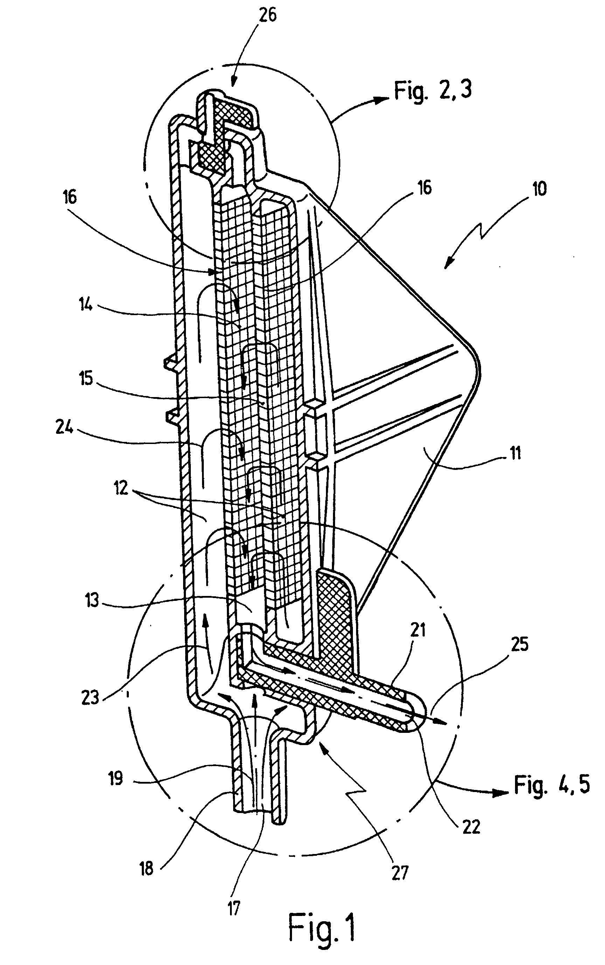

In Fig. 1 ist in einem perspektivischen Längsschnitt eine Vorrichtung 10 zur Filtration und Entgasung von Körperflüssigkeit gezeigt, die in dem gewählten Ausführungsbeispiel ein Blutfilter ist. Die Vorrichtung 10 ist als dead-end-Filter ausgebildet, so daß im Gegensatz zu cross-flow-Filtern Blutverlust im Einsatz vermieden wird.

Die Vorrichtung 10 umfaßt ein prismatisches Gehäuse 11, in dem eine erste Filterkammer 12 sowie eine zweite Filterkammer 13 angeordnet sind.

Die zweite Filterkammer 13 ist von zwei Seitenwänden 14, 15 begrenzt, die jeweils mit einem Filtermedium 16 bestückt sind, das die erste Filterkammer 12 von der zweiten Filterkammer 13 trennt.

An ihrem in der in Fig. 1 gezeigten Betriebsstellung tiefsten Punkt weist die Vorrichtung 10 einen Einlaß 17 an einem Zulaufanschluß 18 auf, durch den ein bei 19 angedeuteter Körperflüssigkeitsstrom in die erste Filterkammer 12 hinein gelangt. Unmittelbar benachbart zu dem Zulaufanschluß 19 befindet sich ein Ablaufanschluß 21 mit einem Auslaß 22.

Der in die Vorrichtung 10 hinein gelangende Körperflüssigkeitsstrom 19 steigt in der ersten Filterkammer 12 nach oben, wie es durch einen Pfeil 23 angedeutet ist, und tritt seitlich durch das Filtermedium 16 hindurch in die zweite Filterkammer 13, was durch Pfeile 24 angezeigt ist. In der zweiten Filterkammer 13 sinkt die Körperflüssigkeit nach unten und tritt aus dem Auslaß 22 als filtrierte Körperflüssigkeit wieder aus, was durch einen Pfeil 25 angedeutet ist.

An ihrem oberen Ende weist die Vorrichtung 10 eine gemeinsame Be- und Entlüftungseinrichtung 26 für die erste und die zweite Filterkammer 12, 13 auf, wobei an ihrem von dem oberen Ende abgelegenen unteren Ende zwischen dem Zulaufanschluß 18 sowie dem Ablaufanschluß 21 ein Verbindungselement 27 vorgesehen ist.

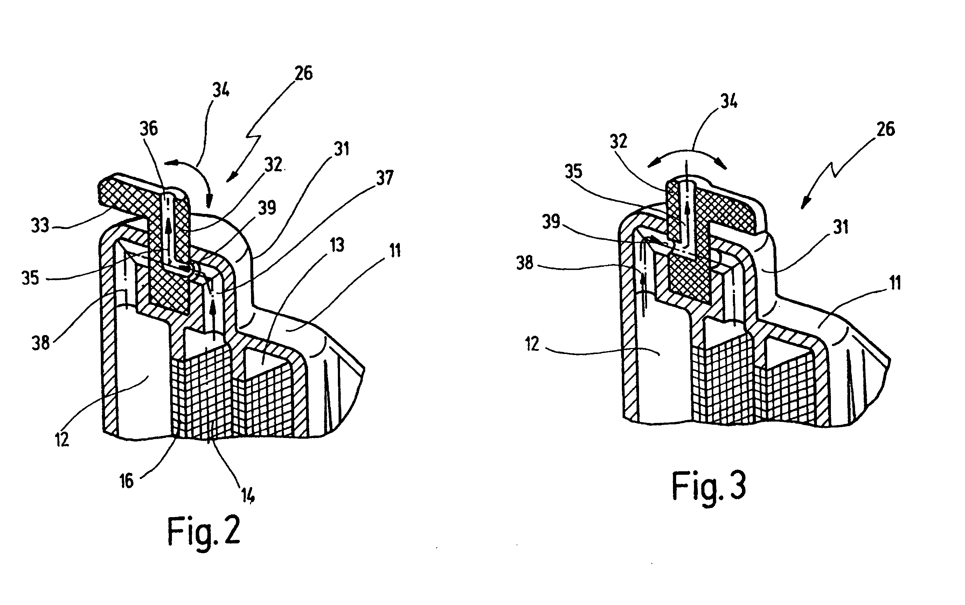

In den Figuren 2 und 3 ist die gemeinsame Be- und Entlüftungseinrichtung 26 in einem vergrößerten Längsschnitt gezeigt. Die Be- und Entlüftungseinrichtung 26 ist an einer oberen Ecke 31 des Gehäuses 11 angeordnet und umfaßt einen in dem Gehäuse 11 drehbar gelagerten Hohlzylinder 32, der über einen Hebel 33 in Richtung eines Pfeiles 34 verschwenkt werden kann.

In dem Hohlzylinder 32 ist ein seitlich offener Innenkanal 35 vorgesehen, dessen nach oben offener Auslaß 36 zum Gasaustritt dient.

Von der zweiten Filterkammer 13 führt ein nach oben weisender Steigkanal 37 und von der ersten Filterkammer 12 ein nach oben weisender Steigkanal 38 zu dem Hohlzylinder 32, dessen seitliche Öffnung 39 bei dem Betriebszustand der Fig. 2 mit dem Steigkanal 37 und bei dem Betriebszustand der Fig. 3 mit dem Steigkanal 38 kommuniziert.

Auf diese Weise ist es möglich, durch einfaches Verdrehen des Hebels 33 zunächst beim Befüllen der Vorrichtung 10 mit Körperflüssigkeit die erste Filterkammer 12 und dann die zweite Filterkammer 13 zu entlüften, wobei in einer dritten, in den Figuren nicht gezeigten Betriebsstellung der Hebel 33 eine Stellung um 90° zwischen den in den Figuren 2 und 3 gezeigten Stellungen einnimmt, so daß die Öffnung 39 verschlossen ist und keine Körperflüssigkeit aus dem Auslaß 36 mehr austreten kann.

Durch die einfache Konstruktion der Be- und Entlüftungseinrichtung 26 ist nicht nur eine einfache Handhabung sondern auch eine einfache Reinigung und Sterilisation möglich.

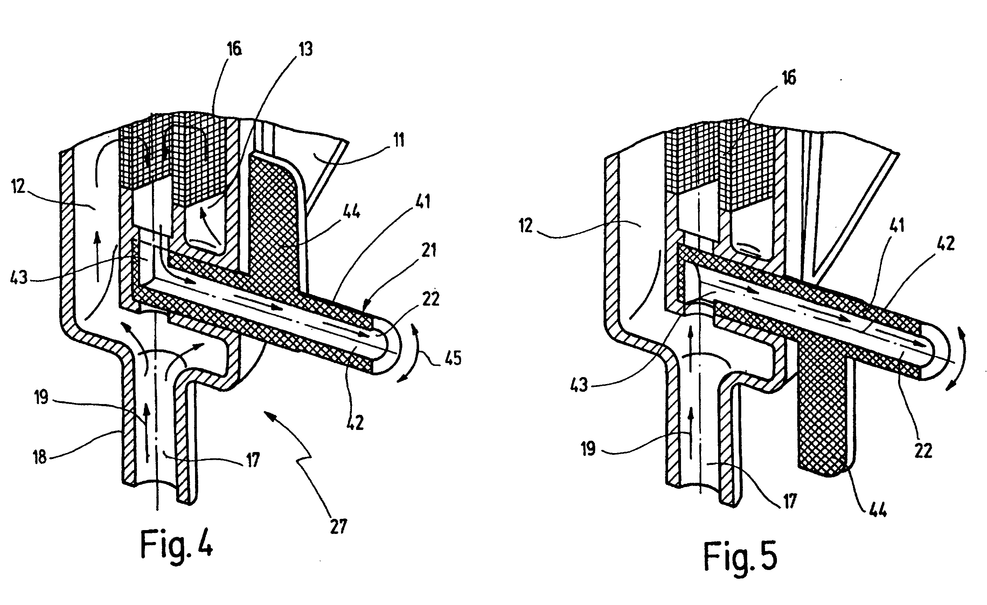

In den Figuren 4 und 5 ist das an dem unteren Ende des Gehäuses 11 gelegene Verbindungselement 27 gezeigt, das ein in dem Gehäuse 11 drehbar gelagertes Rohrstück 41 umfaßt, dessen Bohrung 42 innen im Gehäuse 11 in eine seitlich abgehende Öffnung 43 mündet.

An dem Rohrstück 41 ist ein Hebel 44 befestigt, durch den das Rohrstück 41 um 180° schwenkbar ist, wie es durch einen Pfeil 45 angedeutet ist.

In der in Fig. 4 gezeigten Betriebsstellung ist die Öffnung 43 mit der zweiten Filterkammer 13 in Verbindung, so daß gefilterte Körperflüssigkeit in die Bohrung 42 und von dort zu dem Auslaß 22 gelangen kann.

Bei der anderen Betriebsstellung gemäß Fig. 5 ist die Öffnung 43 dagegen unmittelbar mit dem Einlaß 17 verbunden, so daß unter Umgehung des Filtermediums 16 der Körperflüssigkeitsstrom 19 unmittelbar aus dem Auslaß 22 wieder austritt.

Durch diesen "integrierten Bypass" ist es möglich, ohne weitere Bypassschläuche oder Verbindungsklemmen die Vorrichtung 10 wahlweise in einen extrakorporalen Blutkreislauf einzuschalten oder das Filtermedium sozusagen kurzzuschließen, wenn es verstopft ist oder die Filterfunktion nicht mehr benötigt wird.

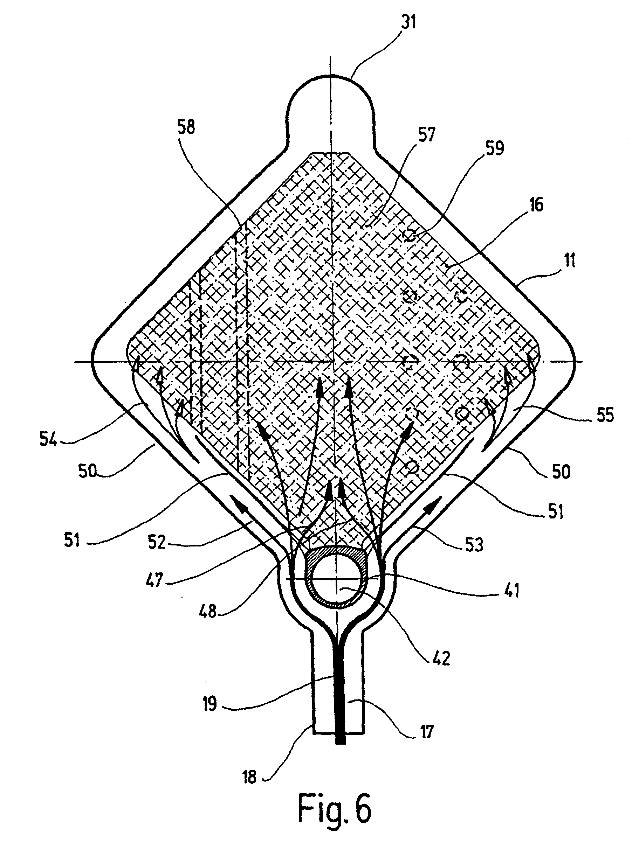

Aus den Figuren 4 und 5 ist noch zu erkennen, daß sich das Rohrstück 41 quer über dem Zulaufanschluß 18 derart erstreckt, daß es unmittelbar in dem Körperflüssigkeitsstrom 19 liegt und diesen in Teilströme aufteilt, wie es besser in der geschnittenen Draufsicht gemäß Fig. 6 zu erkennen ist.

Die durch das Rohrstück 41 erzeugten Teilströme 47 und 48 gehen links und rechts an dem Rohrstück 42 vorbei und strömen das Filtermedium 16 in Fig. 6 von unten an. Das Gehäuse 11 weist gemäß Fig. 6 einen quadratischen Grundriß auf, wobei an seinen beiden unteren Schmalseiten 50 zwei Strömungsbleche 51 vorgesehen sind, die Teilströme 52 und 53 seitlich nach oben leiten, wo sie als Teilströme 54 und 55 etwa mittig auf das Filtermedium 16 treffen.

Das Rohrstück 41 sowie die Strömungsbleche 51 bilden sozusagen Strömungsleitmittel, über die der Körperflüssigkeitsstrom 19 in verschiedene Teilströme aufgeteilt wird, die das Filtermedium 16 teilweise von unten und teilweise seitlich etwa mittig anströmen, wobei weitere, in Fig. 6 nicht gezeigte Teilströme entstehen, die noch weiter oben auf das Filtermedium 16 einströmen.

Durch diese Verteilung der Teilströme wird eine Verwirbelung der Sedimente erreicht, so daß sich das Filtermedium 16 nicht - wie es im Stand der Technik erfolgt - langsam von unten nach oben zunehmend zusetzen kann.

Das Filtermedium 16 ist ein Vliesgewebe 57, das auf vertikal ausgerichteten Stegen 58 oder Noppen 59 aufliegt, die als Stützelemente für das Vliesgewebe 57 dienen und die lichte Weite der zweiten Filterkammer 13 bestimmen.

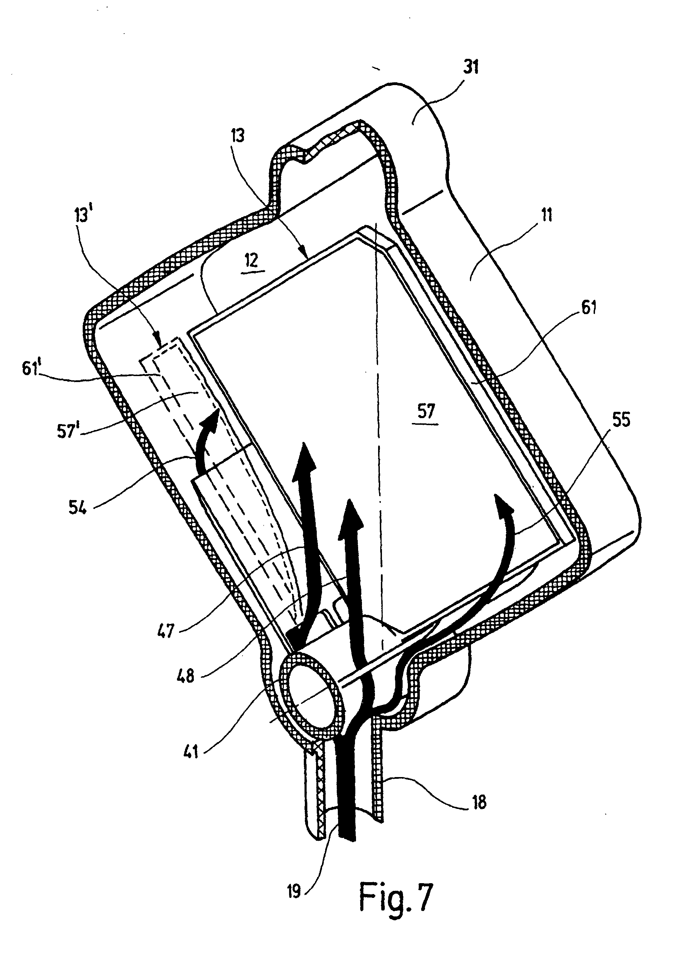

Zur Vergrößerung der Filterfläche ist es möglich, statt einer zweiten Filterkammer 13 mehrere parallel zueinander angeordnete, flach ausgebildete Filterkammern 13, 13' einzusetzen, wie es in Fig. 7 angedeutet ist. Jede einzelne Filterkammer 13, 13' umfaßt dabei einen Tragrahmen 61, 61', auf dem jeweils das Vliesgewebe 57 aufgespannt ist, wobei es durch die aus Fig. 6 bekannten Stützelemente 58 oder 59 unterstützt wird, um über der gesamten Fläche die lichte Weite der zweiten Filterkammer 13, 13' beizubehalten.

In Fig. 7 ist noch einmal die Verteilung der Teilströme 47, 48, 54, 55 gezeigt, die für eine effektive Ausnutzung der gesamten Fläche des Filtermediums 16 sorgen.

## Patentansprüche

1. Vorrichtung zur Filtration und Entgasung von Körperflüssigkeit (19), insbesondere Blutfilter, mit zumindest zwei voneinander durch ein Filtermedium (16) getrennten Filterkammern (12, 13), die vorzugsweise in einem gemeinsamen Gehäuse (11) angeordnet sind, wobei die erste Filterkammer (12) mit einem Zulaufanschluß (18) für zu filtrierende Körperflüssigkeit (19) und die zweite Filterkammer (13) mit einem Ablaufanschluß (21) für filtrierte Körperflüssigkeit verbunden ist,
**dadurch gekennzeichnet, daß** eine beiden Filterkammern (12, 13) gemeinsame Be- und Entlüftungseinrichtung (26) vorgesehen ist, die wahlweise und separat mit der ersten bzw. zweiten Filterkammer (12, 13) verbindbar ist.

2. Vorrichtung nach Anspruch 1, **dadurch gekennzeichnet, daß** zwischen dem Zulauf- und dem Ablaufanschluß (18, 21), die vorzugsweise zueinander benachbart am in Betriebsstellung tiefsten Punkt der Vorrichtung (10) angeordnet sind, ein Verbindungselement (27) vorgesehen ist, über das Zulaufund Ablaufanschluß (18, 21) unter Umgehung des Filtermediums (16) wahlweise direkt miteinander verbindbar sind.

3. Vorrichtung nach Anspruch 1 oder 2, **dadurch gekennzeichnet, daß** in der ersten Filterkammer (12) Strömungsleitmittel (41, 51) vorgesehen sind, die die eintretende Körperflüssigkeit (19) in zumindest einen ersten, das Filtermedium (16) in Betriebsstellung von unten und zumindest einen weiteren, das Filtermedium (16) etwa mittig anströmenden Teilstrom (47, 48, 54, 55) aufteilen.

4. Vorrichtung nach einem der Ansprüche 1 bis 3, **dadurch gekennzeichnet, daß** das Gehäuse eine prismatische Form mit vorzugsweise quadratischem Querschnitt aufweist und an einer Ecke (31) des Gehäuses (11) die Be- und Entlüftungseinrichtung (26) angeordnet ist.

5. Vorrichtung nach einem der Ansprüche 1 bis 4, **dadurch gekennzeichnet, daß** die Be- und Entlüftungseinrichtung (26) einen drehbar gelagerten Hohlzylinder (32) umfaßt, dessen nach außen führender Innenkanal (35) durch Drehen des Hohlzylinders (32) wahlweise mit einer der beiden Filterkammern (12, 13) verbindbar ist.

6. Vorrichtung nach einem der Ansprüche 1 bis 5, **dadurch gekennzeichnet, daß** der Ablaufanschluß (21) ein Rohrstück (41) umfaßt, das sich in der ersten Filterkammer (12) vorzugsweise als Strömungsleitmittel quer über den Zulaufanschluß (18) erstreckt.

7. Vorrichtung nach Anspruch 6, **dadurch gekennzeichnet, daß** das Rohrstück (41) drehbar gelagert ist und eine nach außen führende Bohrung (42) aufweist, deren innere Öffnung (43) durch Drehen wahlweise mit der zweiten Filterkammer (13) oder dem Zulaufanschluß (18) verbindbar ist, so daß das Rohrstück (41) als Verbindungselement (27) wirkt.

8. Vorrichtung nach einem der Ansprüche 1 bis 7, **dadurch gekennzeichnet, daß** die zweite Filterkammer (13) zumindest zwei parallele, flach ausgebildete Teilkammern (13, 13') umfaßt, die in der ersten Filterkammer (12) angeordnet sind und von dieser beidseitig durch Filtermedium (16) getrennt sind.

9. Vorrichtung nach einem der Ansprüche 1 bis 8, **dadurch gekennzeichnet, daß** das Filtermedium (16) als Vlies- oder Siebgewebe (57) ausgebildet und so an Tragplatten oder Tragrahmen (62) befestigt ist, daß keine Gasblasen eingefangen werden.

10. Vorrichtung nach einem der Ansprüche 1 bis 9, **dadurch gekennzeichnet, daß** das Filtermedium (16) durch in Betriebsstellung der Vorrichtung (10) vertikal ausgerichtete Stützelemente, vorzugsweise Stege (58) oder Noppen (59), unterstützt wird.

11. Vorrichtung nach einem der Ansprüche 3 bis 10, **dadurch gekennzeichnet, daß** die Strömungsleitmittel Strömungsbleche (51) umfassen, die parallel zu Schmalseiten (50) des Gehäuses (11) angeordnet sind.

## Claims

1. An apparatus for filtering and degassing body fluid (19), in particular a blood filter, having at least two filter chambers (12, 13) separated from each other by a filter medium (16), said filter chambers being preferably arranged in a common housing, whereby said first filter chamber (12) is connected to an inflow connector (18) for body fluid (19) to be filtered, and the second filter chamber (13) is connected to an outflow connector (21) for filtered body fluid,
**characterized in that**
an aeration and venting device (26), common to both filter chambers (12, 13), is provided that is connectable selectably and separately to the first and second filter chamber (12, 13), respectively.

2. The apparatus of claim 1, **characterized in that** between said inflow and outflow connector (18, 21), which are preferably arranged adjacent to one another at a point of the apparatus (10) that is lowest in its operation position, a connecting element is arranged by which said inflow and outflow connector (18, 21) are selectably directly inter-connectable by bypassing said filter medium (16).

3. The apparatus of claim 1 or 2, **characterized in that** in said first filter chamber (12), flow directing means (41, 51) are provided that divide inflowing body fluid (19) into at least a first sub-flow (47, 48, 54, 55) flowing against said filter medium (16) in the operating position from below, and at least a further sub-flow flowing against said filter medium (16) approximately centeredly.

4. The apparatus of anyone of claims 1 - 3, **characterized in that** said housing has a prismatic shape with preferably square cross sections, and that at one corner (31) of said housing (11), said aeration and venting device (26) is arranged.

5. The apparatus of anyone of claims 1 - 4, **characterized in that** the aeration and venting device (26) comprises a rotatably mounted hollow cylinder (32) whose outward leading inner channel (35) is selectably connectable to one of said two filter chambers (12, 13) by rotating said hollow cylinder (32).

6. The apparatus of anyone of claims 1 - 5, **characterized in that** said outflow connection (51) comprises a tube segment (41) that extends in said first filter chamber (12), preferably as a flow directing means transversally above said inflow connector (18).

7. The apparatus of claim 6, **characterized in that** said tube segment (41) is rotatably mounted and has an outward-leading bore (42) whose inner opening (43) is selectably connectable, by rotation, to said second filter chamber (13) and said inflow connector (18), respectively, such that said tube segment (41) acts as a connecting element (27).

8. The apparatus of anyone of claims 1 - 7, **characterized in that** said second filter chamber (13) comprises at least two parallel sub-chambers (13, 13') that are configured flat and are arranged in said first filter chamber (12) and separated therefrom on both sides by filter medium (16).

9. The apparatus of anyone of claims 1 - 8, **characterized in that** said filter medium (16) is configured as a non-woven fabric or sieve fabric (57) and attached to supporting plates of frames (62) such that no gas bubbles are captured.

10. The apparatus of anyone of claims 1 - 9, **characterized in that** said filter medium (16) is supported by support elements, preferably webs (58) or knobs (59), that are aligned vertically when the apparatus (10) is in the operating position.

11. The apparatus of anyone of claims 3 - 10, **characterized in that** said flow-directing means comprise flow panels (51) that are arranged parallel to narrow sides (50) of said housing (11).

## Revendications

1. Dispositif destiné à la filtration et au dégazage de liquide corporel (19), notamment filtre de sang, comportant au moins deux compartiments de filtration (12, 13) séparés l'un de l'autre par un milieu filtrant (16), qui sont de préférence disposés dans un boîtier (11) commun, le premier compartiment de filtration (12) étant relié à un raccordement d'entrée (18) pour du liquide corporel (19) à filtrer, et le deuxième compartiment de filtration (13) étant relié à un raccordement de sortie (21) pour du liquide corporel filtré,
**caractérisé en ce qu'**il est prévu un dispositif d'aération et de désaération (26) commun aux deux compartiments de filtration (12, 13), qui peut être relié, au choix et séparément, au premier ou au deuxième compartiment de filtration (12, 13).

2. Dispositif selon la revendication 1, **caractérisé en ce qu'**un élément de liaison (27) est prévu entre les raccordements d'entrée et de sortie (18, 21), qui sont de préférence disposés au voisinage l'un de l'autre au niveau du point le plus bas dans la position de fonctionnement du dispositif (10), par l'intermédiaire duquel les raccordements d'entrée et de sortie (18, 21) peuvent, au choix, être reliés directement entre eux avec contournement du milieu filtrant (16).

3. Dispositif selon la revendication 1 ou 2, **caractérisé en ce que** des moyens de guidage d'écoulement (41, 51) sont prévus dans le premier compartiment de filtration (12), qui répartissent le liquide corporel (19) entrant en au moins un premier courant partiel (47, 48, 54, 55) affluant au milieu filtrant (16) à partir du bas dans la position de fonctionnement, et en au moins un autre courant partiel affluant au milieu filtrant (16) sensiblement au centre.

4. Dispositif selon l'une quelconque des revendications 1 à 3, **caractérisé en ce que** le boîtier présente une forme prismatique avec une section transversale qui est de préférence quadratique, et **en ce que** le dispositif d'aération et de désaération (26) est placé dans un angle (31) du boîtier (11).

5. Dispositif selon l'une quelconque des revendications 1 à 4, **caractérisé en ce que** le dispositif d'aération et de désaération (26) comporte un cylindre creux (32) monté en rotation, dont le canal intérieur (35) conduisant vers l'extérieur peut, au choix, être relié à l'un des deux compartiments de filtration (12, 13) par la rotation du cylindre creux (32).

6. Dispositif selon l'une quelconque des revendications 1 à 5, **caractérisé en ce que** le raccordement de sortie (21) comporte un élément tubulaire (41), qui s'étend dans le premier compartiment de filtration (12) transversalement au-dessus du raccordement d'entrée (18), de préférence en tant que moyen de guidage d'écoulement.

7. Dispositif selon la revendication 6, **caractérisé en ce que** l'élément tubulaire (41) est monté en rotation et comporte un perçage (42) conduisant vers l'extérieur, dont l'orifice intérieur (43) peut être relié par rotation, au choix, au deuxième compartiment de filtration (13) ou au raccordement d'entrée (18), de sorte que l'élément tubulaire (41) fasse office d'élément de liaison (27).

8. Dispositif selon l'une quelconque des revendications 1 à 7, **caractérisé en ce que** le deuxième compartiment de filtration (13) comporte au moins deux compartiments partiels (13, 13') parallèles agencés de façon plate, qui sont disposés dans le premier compartiment de filtration (12), et qui sont séparés des deux côtés de celui-ci par du milieu filtrant (16).

9. Dispositif selon l'une quelconque des revendications 1 à 8, **caractérisé en ce que** le milieu filtrant (16) est agencé sous la forme d'un non-tissé ou d'une gaze métallique (57), et est fixé sur des plaques de support ou des cadres de support (62) de sorte à ne pas capter des bulles de gaz.

10. Dispositif selon l'une quelconque des revendications 1 à 9, **caractérisé en ce que** le milieu filtrant (16) est soutenu par des éléments de soutien, de préférence des barrettes (58) ou des nopes (59), qui sont orientés verticalement dans la position de fonctionnement du dispositif (10).

11. Dispositif selon l'une quelconque des revendications 3 à 10, **caractérisé en ce que** les moyens de guidage d'écoulement comportent des tôles d'écoulement (51), qui sont disposées parallèlement aux faces étroites (50) du boîtier (11).
